# EUROPEAN PATENT SPECIFICATION

(11) **EP 3 493 779 B1**
(45) Date of publication and mention of the grant of the patent: **18.03.2026**
(21) Application number: 17752253.9
(22) Date of filing: 04.08.2017
(51) Int. Cl.: A61F 11/00

(54) **SYSTEMS FOR DELIVERY OF THERAPEUTIC SUBSTANCE TO THE MIDDLE AND/OR INNER EAR**
SYSTEME ZUR ABGABE EINER THERAPEUTISCHEN SUBSTANZ AN DAS MITTEL- UND/ODER INNENOHR
SYSTÈMES D'ADMINISTRATION DE MÉDICAMENT POUR L'OREILLE MOYENNE ET/OU INTERNE.

(30) Priority: 05.08.2016 US 201662371583 P
(43) Date of publication of application: 12.06.2019
(73) Proprietor: Tusker Medical, Inc., Austin, TX 78735 (US)
(72) Inventor: GOLDFARB, Eric, Belmont, California 94002 (US); GIROTRA, Rohit, San Francisco, California 94131 (US); ABOLFATHI, Amir, Woodside, California 94062 (US)
(74) Representative: Leach, Sean Adam
(86) International application number: PCT/US2017/045438
(87) International publication number: WO 2018/027102

(56) References cited:
- EP-A1- 1 415 671
- EP-A2- 1 424 050
- EP-B1- 1 133 269
- WO-A1-2014/160398
- WO-A1-2015/168642
- WO-A2-2011/019954

## Description

### TECHNICAL FIELD

The present disclosure relates generally to systems, apparatus, and methods for delivering a substance to an ear of a subject. More specifically, the present disclosure relates to systems, apparatus, and methods for accessing and delivering a therapeutic substance to the subject's tympanic cavity or middle ear and/or inner ear.

### BACKGROUND

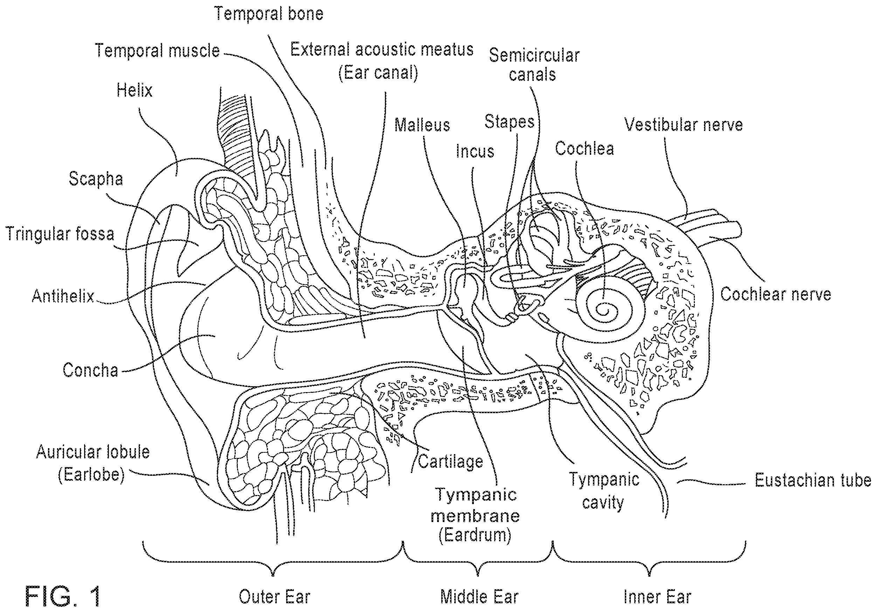

In humans and many other animals, the tympanic cavity is a small cavity in the petrous temporal bone. As shown in FIG. 1, the tympanic cavity surrounds the bones of the middle ear, including the malleus, incus, and stapes, and is bounded by six walls. The convex lateral wall, which separates the tympanic cavity from the external auditory canal and outer ear, includes the tympanic membrane. The convex medial wall, which separates the tympanic cavity from the inner ear, includes the oval window, round window, and the promontory formed by the first turn of the cochlea. The roof separates the tympanic cavity from the cranial cavity, the floor separates the tympanic cavity from the jugular vein, the posterior wall separates the tympanic cavity from the mastoid antrum air cells, and the anterior wall separates the tympanic cavity from the carotid canal.

The tympanic cavity is hollow and normally filled with air; however, the air of the tympanic cavity usually is not in direct contact with the atmosphere of the outside environment. When a pressure difference develops between the tympanic cavity and the outside environment (e.g., because the subject moves between altitudes as on an airplane or dives into water), the tympanic membrane may become damaged if it is not relieved. If tympanic cavity pressure remains lower than the atmosphere, the tympanic membrane may retract into the tympanic cavity. If tympanic cavity pressure remains higher than the atmosphere, the tympanic membrane may rupture.

Otitis media is an inflammation of the middle ear (without reference to etiology or pathogenesis) and is particularly common in human children due to their anatomy and immune function. If severe or untreated, otitis media may result in rupture of the tympanic membrane, hearing loss, or intracranial complications. Otitis media may be managed using oral and topical pain killers (e.g.. ibuprofen. acetaminophen, opiates, antipyrine, benzocaine ear drops) and antibiotics (e.g., amoxicillin, amoxicillin-clavulanate, beta lactamase inhibitor).

The Eustachian tube helps equalize middle ear pressure and drain fluid from the middle ear by connecting the tympanic cavity to the nasopharynx; however, Eustachian tubes are susceptible to inflammation and dysfunction.

To relieve pressure or pain from pressure differences and/or inflammation, the tympanic membrane may be punctured (e.g., by performing a myringotomy, tympanostomy, or tympanocentesis). Fluid present in the middle ear may be aspirated during a procedure. In some procedures, a tube or grommet is inserted into the tympanic membrane to drain fluid from the tympanic cavity and/or to provide a path for equalization of pressure.

The footplate of the stapes in the middle ear connects to the oval window, a membrane-covered opening that leads from the middle ear to the inner car, which is a labyrinth of fluid-filled tubes and sacs, including the vestibule, semicircular canals, and cochlea. When the stapes presses on the oval window, the fluid (e.g., perilymph) of the inner ear moves, thus converting energy from mechanical vibrations to waves in the fluid and detecting hair cells of the inner ear. The round window is a second opening between the middle and inner ear covered by a membrane, which vibrates with opposite phase to vibrations entering the inner ear through the oval window. The cochlea converts and transmits sound signals to the brain, while the vestibular system (i.e., the vestibule and semicircular canals) provides the brain with information about head turns, translations, and tilts used for balance, spatial orientation, and motion.

Vestibular neuritis and labyrinthitis are inflammations of the inner ear (without reference to etiology or pathogenesis) caused by viral infections, bacterial infections, autoimmune disorders, and/or physical blockage of the inner ear resulting in symptoms that include dizziness, vertigo, nausea, tinnitus, and hearing changes. Neuritis and labyrinthitis may be managed using oral and topical (via, e.g., transtympanic perfusion and cochlear microperfusion) antioxidants, antiviral agents, anti-inflammatories, immunosuppressive agents, and antibiotics (e.g., broad-spectrum antibiotics or combination therapy with central nervous system penetration).

In addition to inflammation and infection, both the middle and inner ear are susceptible to other disorders including, but not limited to, otosclerosis or otospongiosis (abnormal bone growth near the middle ear), which may be managed using sodium fluoride or bisphosphonates; and Meniere's disease, which may be treated with, for example, gentamicin as part of a chemical labyrinthectomy.
WO 2011/019954 discusses intracochlear drug delivery to the central nervous system.
WO 2014/160398 discusses a trans-tympanic antioxidant delivery system.
WO 2015/168642 discusses a drug-releasing device usable in mucosal body cavities.
EP 1415671 discusses biodegradable drains for medical applications.
EP 1 133 269 B1 describes a drug delivery unit for delivering therapeutic agents to a subject's inner ear. In one example, a hollow elongate member extends from a reservoir containing therapeutic fluid, through an incision in the subject's tympanic membrane, to a drug delivery unit for delivery to the round window of the patient's ear.

### SUMMARY

The present invention is set out in the appended independent claim. Optional features are set out in the appended dependent claims. In the following, any examples and embodiments not falling within the scope of the claims do not form part of the invention and are provided for illustrative purposes only. Any methods for treatment of the human or animal body by therapy described herein do not form part of the invention.

Systems, apparatus, and methods are described for delivering a therapeutic substance to an ear of a subject. In one embodiment, a reservoir is configured to contain the therapeutic substance and an outlet is configured to transport the therapeutic substance from the reservoir to a lumen of a tympanostomy tube disposed within a tympanic membrane bordering the tympanic cavity and/or the tympanic cavity, for example via the lumen of the tympanostomy tube, such that the therapeutic substance can be delivered to the tympanic cavity and/or the round window to the inner ear. The outlet may be a conduit and/or a wick. Alternatively, the outlet may be integral with the tympanostomy tube (e.g., formed as a lumen of tympanostomy tube).

In another embodiment, a tympanostomy tube having a proximal end and a distal end may be configured for deployment in a tympanic membrane bordering the tympanic cavity such that the distal end is positioned in the tympanic cavity. The tympanostomy tube may include a solid composite of a polymer and the therapeutic substance on at least the distal end of the tympanostomy tube such that the therapeutic substance can be eluted in the tympanic cavity and/or to the round window to the inner ear. The solid composite may be an adherent layer, or the tympanostomy tube itself may be formed of the solid composite.

In another embodiment, an apparatus for delivering a therapeutic substance to a tympanic cavity in a subject may include a tympanostomy tube having a proximal end a distal end, and a central lumen therethrough. The tympanostomy tube may be configured for deployment in a tympanic membrane bordering the tympanic cavity such that the distal end is positioned in the tympanic cavity. The apparatus also may include a bladder configured for deployment proximal to the tympanic membrane, storing the therapeutic substance, and having an outlet in fluid communication with the proximal end of the central lumen tympanostomy tube, such that the therapeutic substance can be delivered to the tympanic cavity and/or to the round window to the inner ear via the tympanostomy tube. The apparatus further may include an inlet in fluid communication with the bladder and configured to permit the bladder to be filled with the therapeutic substance when the apparatus is disposed in the ear of the subject.

In another embodiment, a method of delivering a therapeutic substance to a tympanic cavity in a subject includes fonning an incision in a tympanic membrane bordering the tympanic cavity and inserting the therapeutic substance through the incision and into the tympanic cavity. The method may include perfusing the therapeutic substance through a round window to an inner ear bordering the tympanic cavity. The therapeutic substance may be a gas, liquid, colloid, suspension, gel, or solid.

In another embodiment, a method of delivering a therapeutic substance to a tympanic cavity in a subject includes disposing a tympanostomy tube in a tympanic membrane bordering the tympanic cavity and transporting the therapeutic substance from a reservoir to a lumen of a tympanostomy tube disposed within a tympanic membrane bordering the tympanic cavity, the tympanic cavity via the lumen of the tympanostomy tube, and/or a round window bordering the tympanic cavity via the lumen of the tympanostomy tube and the tympanic cavity, such that the therapeutic substance can be delivered to the tympanic cavity and/or inner car. The steps of disposing the tympanostomy tube and transporting the therapeutic substance from the reservoir may occur at about the same time and/or simultaneously.

Other systems, processes, and features will become apparent to those skilled in the art upon examination of the following drawings and detailed description.

### BRIEF DESCRIPTION OF THE DRAWINGS

The skilled artisan will understand that the drawings primarily are for illustrative purposes and are not intended to limit the scope of the inventive subject matter described herein. In addition, it will be appreciated that any examples and embodiments illustrated and described below which do not fall within the scope of the appended claims do not form part of the invention and are provided for illustrative purposes only. Any methods for treatment of the human or animal body by therapy described herein do not form part of the invention. The drawings are not necessarily to scale; in some instances, various aspects of the inventive subject matter disclosed herein may be shown exaggerated or enlarged in the drawings to facilitate an understanding of different features. In the drawings, like reference characters generally refer to like features (c.g., functionally similar and/or structurally similar clements).
FIG. 1 illustrates the anatomy of the human car.
FIG. 2 is a schematic illustration of a therapeutic delivery system according to an cmbodiment.
FIG. 3 is a schematic illustration of a housing for a therapeutic delivery system according to an embodiment.
FIGs. 4A-4C are schematic illustrations of various embodiments of a therapeutic delivery system disposed in the outer car, tympanic membrane, and/or inner car
FIG. 5 is a flow diagram illustrating a method for delivering a therapeutic substance to a tympanic cavity of a subject in accordance with some embodiments.
FIGs. 6A-6C are schematic illustrations of an exemplary tympanostomy tube delivery system,
FIGs. 7A-7B are schematic illustrations of a therapeutic substance delivery system according to an embodiment.
FIGs. 8A-8B are schematic illustrations of a therapeutic substance delivery system according to an embodiment.
FIGs. 9A-9B are schematic illustrations of a therapeutic substance delivery system according to an embodiment.
FIGs. 10A-10B are schematic illustrations of a therapeutic substance delivery system according to an embodiment.
FIGs. 11A-11B are schematic illustrations of a therapeutic substance delivery system according to an embodiment.
FIGs. 12A-12B are schematic illustrations of a therapeutic substance delivery system according to an embodiment.
FIGs. 13A-13B are schematic illustrations of a therapeutic substance delivery system according to an embodiment.
FIGs. 14A-14B are schematic illustrations of a therapeutic substance delivery system according to an embodiment.
FIGs. 15A-15B are schematic illustrations of a therapeutic substance delivery system according to an embodiment.

### DETAILED DESCRIPTION

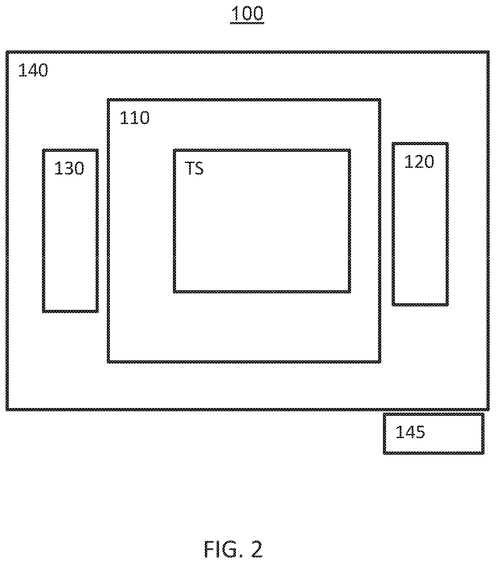

Systems, apparatus, and methods are described herein for delivery of therapeutic substances to the middle car, and in particular to the tympanic cavity. As illustrated schematically in FIG. 2, a delivery system 100 can include a reservoir 110 that can contain a therapeutic substance TS. An outlet 120 can be coupled fluidically to reservoir 110, enabling therapeutic substance TS to enter the tympanic cavity TC. An inlet 130 can be coupled fluidically to reservoir 110, enabling reservoir 110 to be filled, or refilled, with therapeutic substance TS. Delivery system 100 can include a housing 140 that supports, or defines, reservoir 110, outlet 120, and/or inlet 130. Delivery system 100 may also include a retaining element 145 by which system 100 can be engaged with and selectively retained in a desired location and/or orientation on the subject in operative relationship to the inner ear, e.g., the outer ear OE, the tympanic membrane TM, the round window RW, or some other portion of the anatomy.

Each component of delivery system 100 can be implanted in various ways. Reservoir 110 can be, for example a hollow structure defining a volume within which therapeutic substance TS can be contained. It may be formed as a bladder or balloon with an elastic wall from which therapeutic substances TS in fluid (liquid or gas form) may be expelled by the pressure imposed by the elastic wall of the structure. In this implementation, the delivery rate of therapeutic substance can be controlled by including a suitable orifice in outlet 120. Alternatively. reservoir 110 may include a more rigid-walled hollow structure, from which therapeutic substance TS is expelled by a separate source of pressure, such as pump, syringe (actuated manually, mechanically, pneumatically, etc.) or other device. Alternatively, other forces or mechanisms may be employed to expel, release, or extract therapeutic substance TS from reservoir 110 implemented as a rigid container, such as capillary forces via a small orifice or wick, gravity, etc.

Reservoir 110 may be formed by a solid structure within which therapeutic substance TS (in fluid and/or solid form) may be eluted, diffused, or released by other mechanisms. For example, reservoir 110 may biodegrade in, or into, tympanic cavity TC, releasing therapeutic substance TS as reservoir 110 degrades.

Reservoir 110 may be formed in whole or in part by outer ear OE. For example, therapeutic substance TS in liquid may be deposited into outer ear OE at or near tympanic membrane, and conveyed through incision IN in tympanic membrane TM via, for example, a dedicated tube, a tympanostomy tube TT, and/or a wicking device that conveys therapeutic substance TS by capillary action. Similarly, reservoir 110 may be formed in whole or in part by tympanic cavity TC. For example, therapeutic substance TS in fluid and/or solid form may be introduced into tympanic cavity TC via incision IN in tympanic membrane TM, alone or in combination with delivery of tympanostomy tube TT.

Depending on the implementation of reservoir 110, and its location relative to tympanic cavity TC, delivery system 100 may include various implementations of an outlet 120 to convey therapeutic substance TM into tympanic cavity TC and, in some applications, to round window RW. For example, outlet 120 may be implemented as an elongated tube of suitable length to extend from reservoir 110 to tympanic cavity TC (and further to round window RW). For example, reservoir 110 may be disposed in the outer ear near to, or far from, tympanic membrane TM, or may be external to the ear, e.g. behind the helix of the ear. The tube may be sufficiently larger that gravitational and/or pressure-induced forces may drive therapeutic substance TS through the tube, or may be small enough for capillary forces to convey therapeutic substance TS to tympanic cavity TC (and further to round window RW).

If delivery system 100 includes an inlet 130 fluidically coupled to reservoir 110 to permit reservoir 110 to be filled, or refilled, with therapeutic substance TS, inlet 130 may be implemented in various ways depending on the implementation, and location relative to tympanic cavity TC, of reservoir 100. For example, inlet 130 may include a one-way valve through which therapeutic substance TS may be introduced into reservoir 110.

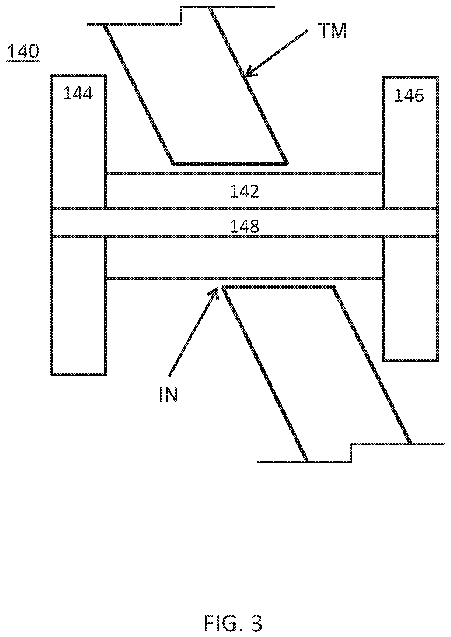

A housing 140 may be also be implemented in various ways depending on the implementation of the other components of delivery system 100 and the location of those components relative to tympanic cavity TC. For example, as shown in FIG. 3, housing 140 may be in a form similar to a conventional tympanostomy tube TT, e.g. with a medial portion 142 sized to be disposed in incision IN through tympanic membrane TM, a distal portion 144 disposed on the tympanic cavity side of tympanic membrane TM, a proximal portion 146 disposed on the outer ear side of tympanic membrane TM, and a lumen 148 therethrough that provides fluid communication between distal portion 144 and proximal portion 146, and thus between outer ear OE and tympanic cavity TC when housing 140 is disposed in incision IN through tympanic membrane TM. Proximal portion 144 and/or distal portion 146 of such a configuration of housing 140 may be formed as a flange, by which housing 140 is inhibited from moving from incision IN into tympanic cavity TC and/or outer ear OE, respectively. (Such flange(s) thus can constitute one or more retaining element(s) 145.) Reservoir 110 may be contained within such an implementation of housing 140, with outlet 120 extending into distal portion 146 of housing 140. Alternatively, reservoir 110 may be formed integrally with housing 140, e.g. in an implementation in which therapeutic substance TS is eluted from, or is released by biodegradation of, a solid reservoir 110. Reservoir 110 may by formed as a portion, or as the entirety, of housing 140. In these implementations, it is not necessary for housing 140 to include lumen 148 therethrough, but, in some implementations, housing 140 can optionally include lumen 148 to provide ventilation of the middle ear. In other implementations, housing 140 includes lumen 148 as with a conventional tympanostomy tube TT, and outlet 120 in the form of a tube or wick may be disposed in fluidic communication with lumen 148, e.g. at the proximal end thereof, of may extend completely through lumen 148 and out the distal end thereof and into tympanic cavity TC.

As discussed above, in some embodiments the delivery system may be in a configuration similar to a tympanostomy tube, and in other embodiments the delivery system may be used in conjunction with a tympanostomy tube. Suitable tympanostomy tubes or tube configurations are disclosed in U.S. Patent No. 9,011,363 to Clopp et al., entitled "Tympanic Membrane Pressure Equalization Tube".

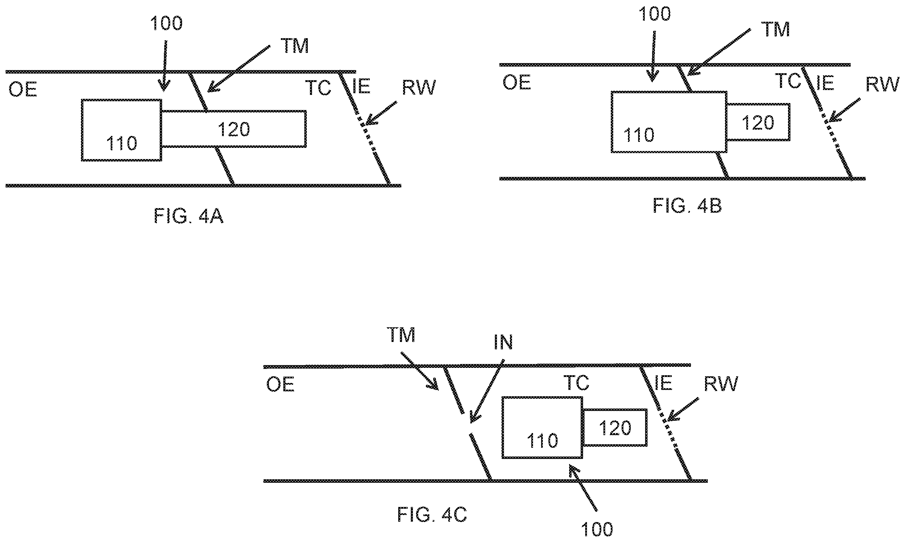

As shown schematically in FIGs. 4A - 4C, delivery system 100, and its constituent components, may be disposed in various positions relative to outer ear OE, tympanic membrane TM, tympanic cavity TC, round window RW, and inner ear IE. For example, in FIG. 4A, reservoir 110 is disposed in outer ear OE, and outlet 120, shown as an elongated structure, extends from reservoir 110 through an incision in tympanic membrane TM and into tympanic cavity TC. In FIG. 4B, reservoir 110 is disposed in an incision in tympanic membrane, and outlet 120 is disposed tympanic cavity TC. In FIG. 4C, reservoir 110 and outlet 120 are disposed in tympanic cavity TC, having been passed through an incision IN in tympanic membrane TM. Outlet 120 may be configured and disposed in close proximity or contact with round window RW.

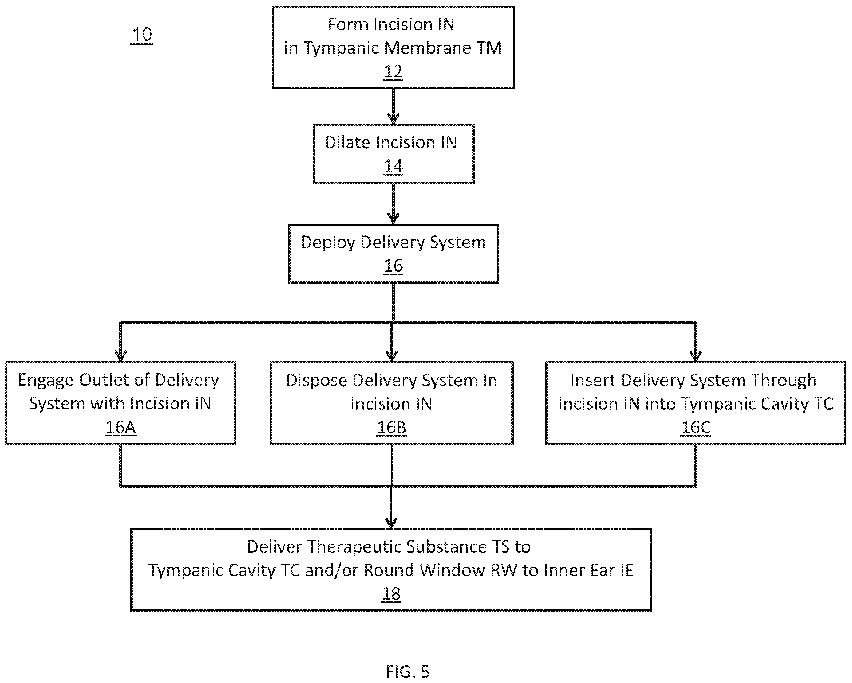

Various methods of delivering therapeutic substance TS to tympanic cavity TC are illustrated schematically in FIG. 5. To reach tympanic cavity TC, an incision IN through tympanic membrane TM is required. Incision IN may already have been created in a separate procedure on the subject, e.g. by a myringotomy or tympanocentesis to enable drainage of medial effusion, or by a tympanostomy in connection with delivery of a tympanostomy tube. Alternatively, formation of incision IN may be performed as an initial step 12 of method 10. Incision IN may be formed using any suitable technique and device known to the artisan.

Incision IN may also be dilated, either as part of a separate procedure, e.g. in connection with delivery of a tympanostomy tube TT, or as step 14 of method 10. The dilation can facilitate insertion of a delivery system 100 as disclosed above. Incision IN may be dilated using any suitable technique and device known to the artisan.

In step 16, a delivery system, such as delivery system 100 disclosed above, is deployed. Deployment may involve, in step 16A, engaging outlet 120 of delivery system 100 with incision IN (or, as disclosed above, with a lumen through a tympanostomy tube TT), as shown in FIG. 4A. Alternatively, in step 16B, deployment of delivery system 100 may involve disposing delivery system 100 into incision IN with outlet 120 in fluidic communication with tympanic cavity TC, as shown in FIG. 4B. Alternatively, in step 16C, deployment of delivery system 100 may involve inserting all or part of delivery system 100 through incision IN into tympanic cavity TC, i.e., so that the entire delivery system 100 is disposed within tympanic cavity TC, as shown in FIG. 4C.

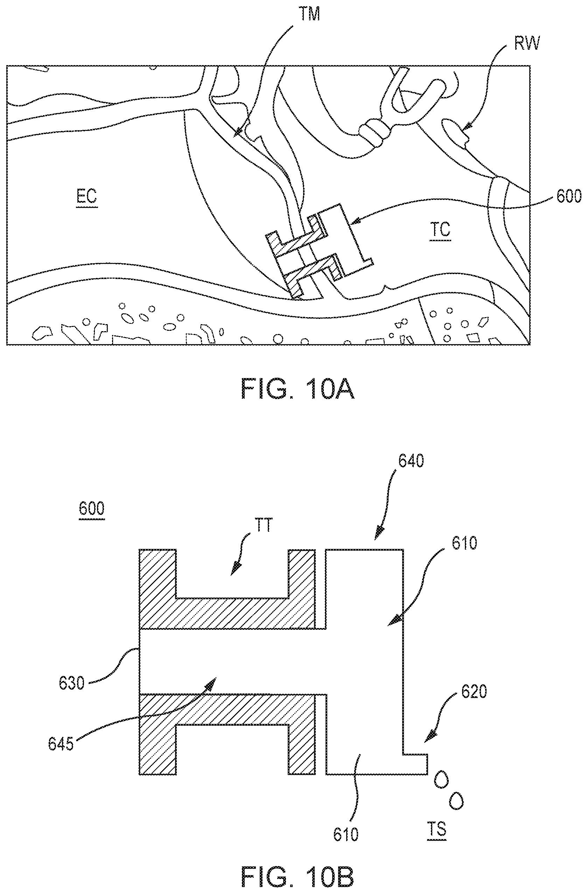

Finally, in step 18, therapeutic substance TS is delivered to tympanic cavity TC and/or inner ear IE via round window RW. Although FIG. 10 illustrates method 10 as occurring in a certain order, the ordering of method 10 may be modified. Additionally, certain of the steps may be performed concurrently in a parallel process when possible, as well as performed sequentially. For example, in some embodiments, step 18 (delivering therapeutic substance TS to tympanic cavity TC and/or round window RW to inner ear IE) can be performed simultaneously with steps 16A, 16B, or 16C.

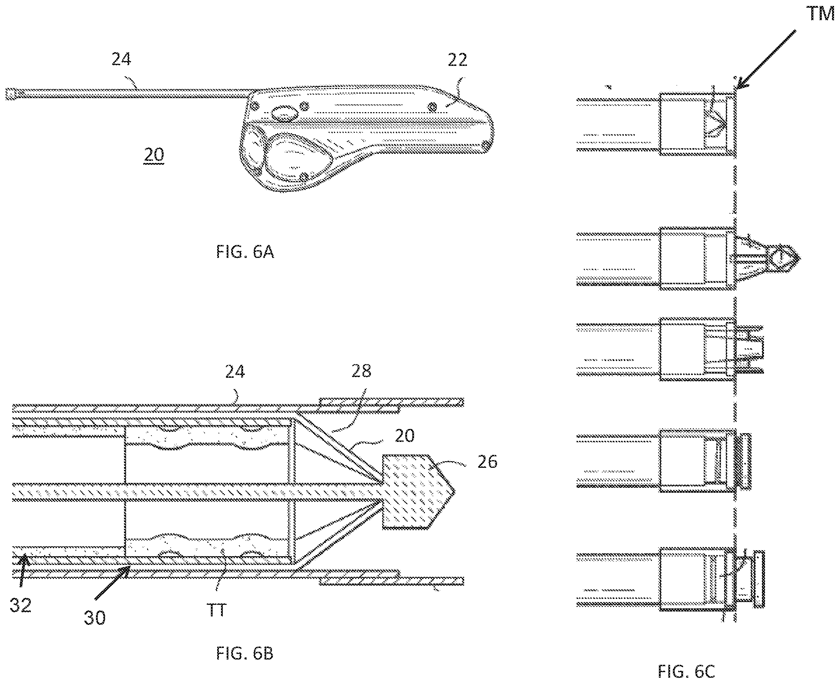

Suitable techniques and devices that may be used to form incision IN (in step 12), to dilate incision IN (in step 14), and/or to deploy delivery system 100 (in step 16) is disclosed in US Patent No. 8,864,774 to Liu et al. entitled "Tympanic Membrane Pressure Equalization Tube Delivery System" (the '774 patent), and U.S. Patent No. 9,681,891 to Andreas et al. entitled "Tympanostomy Tube Delivery Device with Cutting Dilator". As shown in FIG. 6A, tube delivery system (or TDS) 20 includes a housing 22 with a handle and a shaft assembly 24. The distal tip of shaft assembly 24 is shown in cross section in FIG. 6B. As shown in FIG. 6B, shaft assembly 24 includes a cutting member 26 configured to pierce or cut tympanic membrane TM to form incision IN. Shaft assembly 24 also includes a dilator 28 configured to expand within, and thus dilate, incision IN. Shaft assembly 24 also includes a shield 30 and a pusher 32. A tympanostomy tube TT is shown disposed within shield 30. A sequence of operation of TDS 20 is shown in FIG. 6C. Each of the components is selectively driven axially by a cam system, to: a) drive cutting member 26 through tympanic membrane TM to form incision IN; b) retract cutting member 26 and expand dilator 28 within incision IN to dilate incision IN; c) push tympanostomy tube axially through shield 30 with pusher 32 and into incision IN; and d) release tympanostomy tube TT from TDS 20.

As described in more detail below in connection with particular embodiments the TDS described in the '774 patent can be modified to implement particular methods and to deploy particular embodiments of therapeutic substance delivery systems. For example, the TDS can deploy a delivery system 100 into or through tympanic membrane TM instead of, or in addition to, deploying a tympanostomy tube, or can deploy a delivery system in a form similar to a tympanostomy tube. In another modification, the TDS can form incision IN and deposit therapeutic substance TS directly into tympanic cavity TC through incision IN, in any suitable form, including liquid, gel, solid, gas, aerosol, foam, etc. and optionally deploy a tympanostomy tube to be disposed in tympanic membrane TM before, during, or after delivery of therapeutic substance TS. In some embodiments, the TDS deposits therapeutic substance TS in or through a tympanostomy tube disposed in tympanic membrane TM.

Therapeutic substance TS can be any suitable substance or combination of substances, in any suitable dosage form or combination of dosage forms. Non-limiting examples include analgesics (e.g., non-steroidal anti-inflammatory drugs (NSAIDs) like acetaminophen, COX-2 inhibitors, opioids, flupirtine, cannabinoids, capsaicinoids, etc.), anesthetics (e.g. lidocaine, benzocaine, procaine, amethocaine, cocaine, tetracaine, prilocaine, bupivicaine, levobupivacaine, ropivacaine, mepivacaine, dibucaine, etidocaine, etc.), anti-inflammatories (e.g. NSAIDs like aspirin, ibuprofen, and naproxen, peptides, steroids or glucocorticosteroids like dexamethasone, etc.), antibiotics (e.g., ciprofloxacin, ciprofloxacin otic suspension, amoxicillin, amoxicillin-clavulanate, beta lactamase inhibitor, [list]), antivirals, antifungals, antiparasitics, decongestants (e.g., ephedrine, levomethamphetamine, naphazoline, oxymetazoline, phenylephrine, phenylpropanolamine, propylhexedrine, synephrine, tetrahydrozoline, xylometazoline, pseudoephedrine, tramazoline, etc.), mucokinetics (e.g., mucolytics like acetylcysteine, expectorants like guaifenesin, surfactants, etc.), antihistamines, antioxidants, immunosuppressive agents, and dissociatives (e.g., NMDA receptor antagonists like gacyclidine, κ-opioid receptor agonists, etc.). Suitable dosage form(s) can include liquids (including solutions, suspensions, and colloids) delivered (e.g., sprayed) as a liquid, liquid aerosol, foam, emulsion, sol, etc.; gases (including solutions, suspensions, and colloids) delivered as a vapor; and solids (including solutions, suspensions, and colloids) delivered as solid aerosols, solid foam, gel, sol, etc.. including solids that are incorporated into or onto a solid or porous substrate for elution or release by biodegradation of the substrate.

Suitable biodegradable solids may include, but are not limited to, agro-polymers, including polysaccharides and proteins, or biopolyesters including natural monomers (e.g., polyhydroxyalkanoates (PHAs) like poly-3-hydroxybutyrate (PHB), polyhydroxyvalerate (PHV) and polyhydroxyhexanoate (PHH), etc.), renewable monomers (e.g., polylactic acid (PLA)), and synthetic monomers (e.g., polybutylene succinate (PBS), polycaprolactone (PCL), etc.). Additional examples of biodegradable solids include polyglycolic acid, poly(lactic-co-glycolic) acid, poly-e-caprolactone, polydioxanone, chitosan, hyaluronic acid, poly(2-hydroxyethyl-methacrylate), poly(ethylene glycol), polyurethanes, poly(ester amide)s, polyanhydrides, polyvinyl alcohol, cellulose esters, polyethylene terephthalate, and hydrogels. In some embodiments, a normal plastic polymer such as polyethylene or polypropylene may be incorporated with an additive which causes degradation (e.g., due to oxidation). Biodegradable materials may include films, fibers, extruded or molded products, laminates, foams, powders, nonwovens, adhesives, and/or coatings.

Various non-limiting, exemplary embodiments are described below.

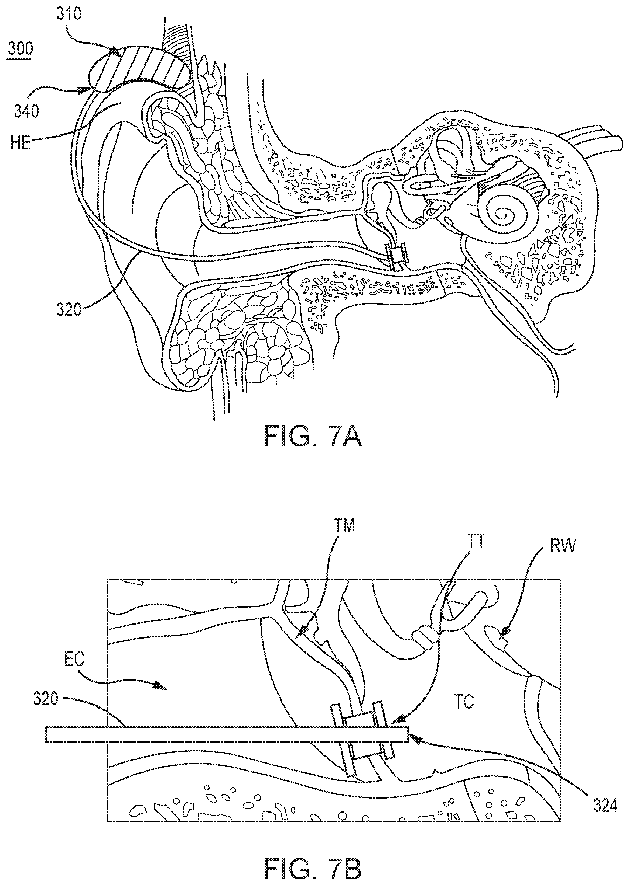

FIGs. 7A and 7B schematically illustrate a delivery system 300 according to a first embodiment. In this embodiment, delivery system 300 includes a reservoir 310 implemented as a hollow container in a housing 340 sized and configured to be disposed behind the ear. e.g. above, behind, and supported on, the helix HE of the car. Outlet 320 is implemented as an elongate tube extending from a proximal end 322 fluidically coupled to reservoir 310, into and through outer ear OE, and into incision IN in tympanic membrane TM. Distal end 324 can be disposed directly in incision IN, in fluidic communication with tympanic cavity TC. Alternatively, as shown in FIG. 7B, distal end 324 can be disposed in the central lumen of tympanostomy tube TT. As discussed above, tympanostomy tube TT may be delivered into incision IN in tympanic membrane TM in a separate procedure, or as part of the method of providing delivery system 300. As described above therapeutic substance TS can be moved from reservoir 310 through outlet 320 with any suitable mechanism, including a pump, gravitational or capillary forces, by pressure exerted by an elastic wall of reservoir 310, etc. Delivery system 300 can be deployed by inserting distal end 324 of outlet 320 through ear canal EC and into and through incision IN or central lumen of tympanostomy tube TT into fluid communication with tympanic cavity TC. Housing 340 can be disposed above, behind, and supported on, for example, the helix HE of the subject's ear.

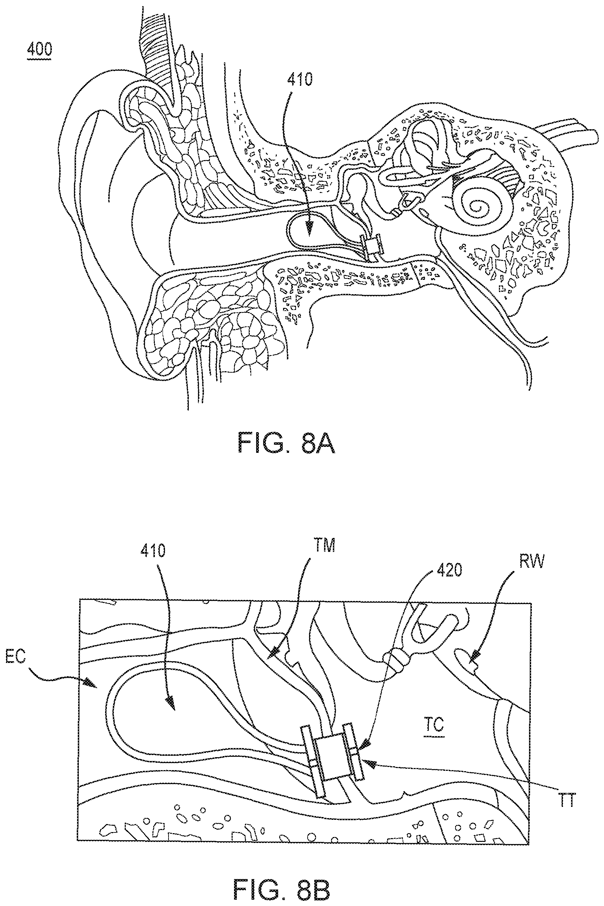

FIGs. 8A and 8B schematically illustrate a delivery system 400 according to another embodiment. In this embodiment, delivery system 400 includes a reservoir 410 disposed within ear canal EC. Reservoir 410 may be implemented as an elastic bladder or balloon, to expel therapeutic substance through outlet 420 into tympanic cavity TC, either via incision IN or the central lumen of tympanostomy tube TT (as shown in FIG. 8B). The flow rate of therapeutic substance TS into tympanic cavity TC may be regulated or limited by an orifice or other flow restriction in outlet 420. Delivery system 400 can be deployed by inserting it through ear canal EC and into position adjacent tympanic membrane TM with outlet 420 inserted into and through incision IN or central lumen of tympanostomy tube TT into fluid communication with tympanic cavity TC.

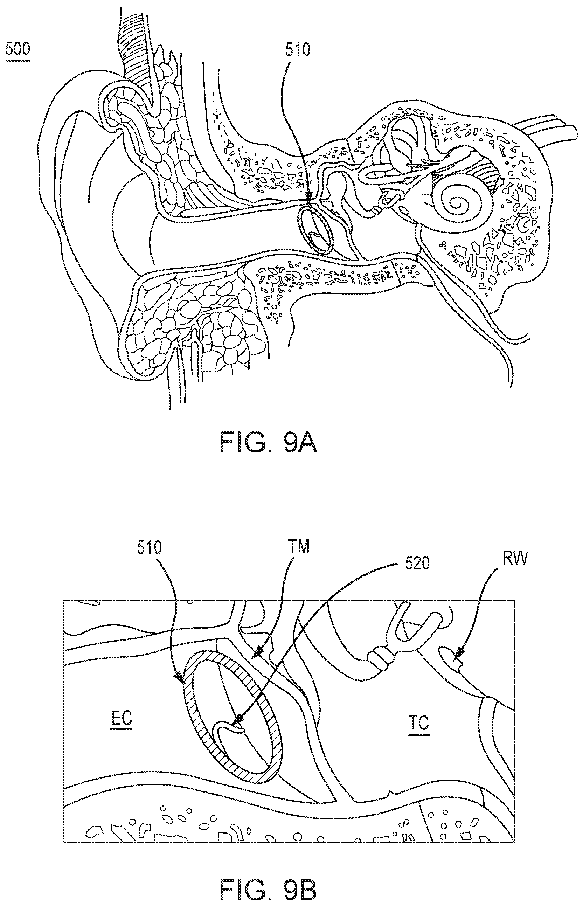

FIGs. 9A and 9B illustrate a delivery system 500 according to another embodiment. Delivery system 500 is similar to delivery system 400, except that reservoir 510 is annular in shape. Delivery system 500 may therefore be disposed near, or in contact with, tympanic membrane TM, and retained in operative proximity to incision IN by anchoring it to ear canal EC, e.g. by resilient expansive force or reservoir 510. Elongate outlet 520 extends from reservoir 510 into and through incision IN in tympanic membrane TM and into tympanic cavity TC, as shown in FIG. 9B. Alternatively, outlet 520 may be disposed through a central lumen of a tympanostomy tube (not shown). Delivery system 500 can be deployed by inserting it through ear canal EC and into position adjacent tympanic membrane TM, and inserting outlet 520 into and through in incision IN in tympanic membrane TM with its distal end in fluid communication with tympanic cavity TC.

In any of the embodiments described above the reservoir of the delivery system can be initially filled, and/or refilled, with therapeutic substance TS after the delivery system has been deployed into the ear of the subject. This may be achieved by including an inlet to the reservoir, implemented, for example, with any suitable one-way valve. Alternatively, the reservoir can be implemented and/or include a wicking material that can convey therapeutic substance TS via capillary action from the outer ear into tympanic cavity TC and/or inner ear IE. In these embodiments, the reservoir can be filled and/or refilled with therapeutic substance TS after deployment by adding therapeutic substance TS to a portion of the wicking material disposed in the outer ear OE or proximate to the outer ear OE. A filling device can be engaged with the inlet and therapeutic substance TS can be discharged from the filling device into the reservoir via the inlet.

FIGs. 10A and 10B are schematic illustrations of a delivery system 600 according to another embodiment. In this embodiment, delivery system 600 can be disposed in, and supported by, a conventional tympanostomy tube TT. Delivery system 600 includes a housing 640 that may be engaged with the central lumen of tympanostomy tube TT. and retained therein by a retaining element 645, e.g. by a frictional fit with the inner wall of the lumen, by adhesive, etc. Reservoir 610 is disposed on a distal end of tympanostomy tube TT, i.e. disposed in the tympanic cavity TC when delivery system is deployed in the subject's ear. Similarly, outlet 620 is disposed in tympanic cavity TC to discharge therapeutic substance TS into tympanic cavity TC. An inlet 630 is disposed on the proximal end of delivery device 600 and tympanostomy tube TT, where it can be accessed via the ear canal. As described above, a filling device can be engaged with inlet 630 and therapeutic substance TS can be discharged from the filling device into reservoir 610 via inlet 630.

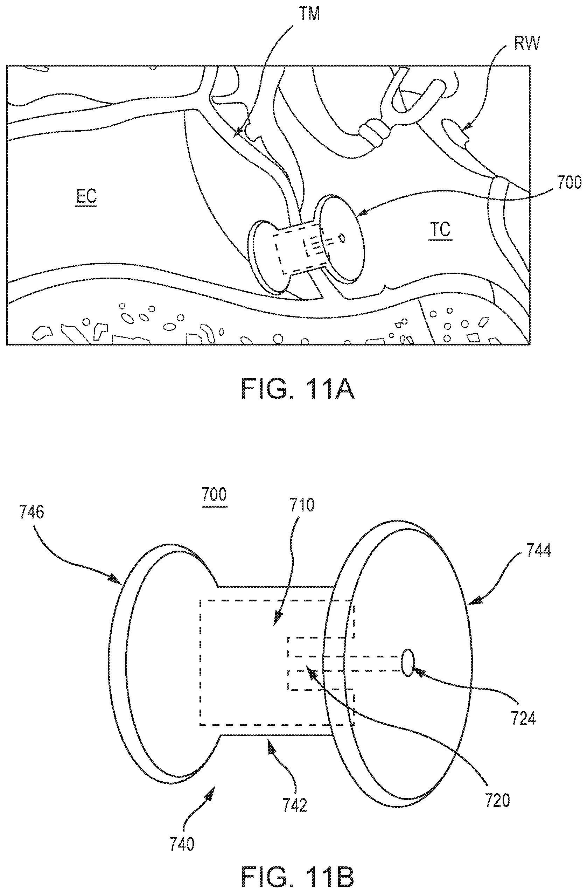

FIGs. 11A and 11B schematically illustrate a delivery system 700 according to another embodiment. In this embodiment, delivery system 700 is configured similar to the form of a conventional tympanostomy tube, with a medial portion 742 sized to be disposed in incision IN through tympanic membrane TM, a distal portion 744 disposed on the tympanic cavity side of tympanic membrane TM, and a proximal portion 746 disposed on the outer ear side of tympanic membrane TM. Delivery system 700 includes a reservoir 710 and an outlet 720 having a distal end 724 through which therapeutic substance TS can be released into tympanic cavity TC. Reservoir 720 may be implemented as a flexible bladder or balloon, and the flow rate of therapeutic substance TS into tympanic cavity TC may be regulated or limited by an orifice or other flow restriction in outlet 720. Delivery system 700 can be deployed by inserting it through ear canal EC and into position in incision IN through tympanic membrane TM with distal end 724 of outlet 720 in fluid communication with tympanic cavity TC. Since, in this embodiment, delivery system 700 is configured similar to the form of a conventional tympanostomy tube, it may be conveniently delivered with the TDS of the '774 patent, optionally including formation and dilation of incision IN in tympanic membrane TM. As with the previous embodiments, reservoir 710 can be filled and/or refilled via an inlet (not shown). In some embodiments, delivery system 700 can include an additional lumen or passageway (not shown) that provides ventilation to the tympanic cavity TC and/or inner ear IE. In other embodiments, reservoir 720 may be formed of a semi-permeable material that provides ventilation of air to the tympanic cavity TC and/or inner ear IE (e.g., via diffusion and/or active transport) but prevents passage of therapeutic substance TS.

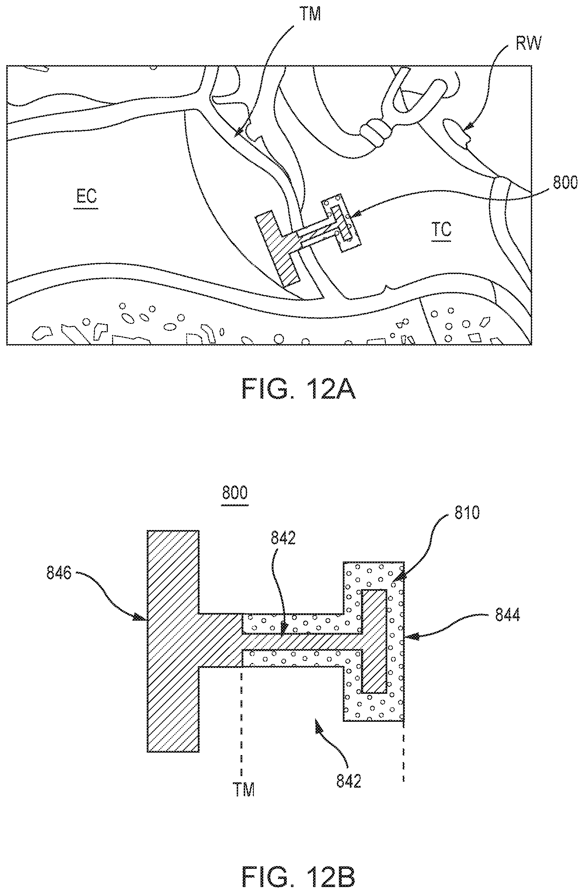

FIGs. 12A and 12B schematically illustrate a delivery system 800 according to another embodiment. As with delivery system 700, delivery system 800 is configured similar to the form of a conventional tympanostomy tube, with a medial portion 842 sized to be disposed in incision IN through tympanic membrane TM, a distal portion 844 disposed on the tympanic cavity side of tympanic membrane TM, and a proximal portion 846 disposed on the outer ear side of tympanic membrane TM. In this embodiment, reservoir 810 is implemented as a solid, or porous, structure rather than as a hollow container. In particular, reservoir 810 is implemented as a layer of therapeutic substance TS in solid form supported on a housing structure 840. Therapeutic substance TS can elute, dissolve, or otherwise release from housing structure 840 into tympanic cavity TC when delivery system 800 is disposed in incision IN in tympanic membrane TM of the ear of the subject. Alternatively, reservoir 810 can be formed of a biodegradable material impregnated with therapeutic substance TS. In a further alternative implementation, the entire delivery system 800 (i.e. the entire structure that is similar to a conventional tympanostomy tube) may be formed of a biodegradable material impregnated with therapeutic substance TS, and/or of a material from which therapeutic substance TS may be eluted. In a further alternative embodiment, a conventional tympanostomy tube, including a central lumen, may be formed of a biodegradable material impregnated with therapeutic substance TS, and/or of a material from which therapeutic substance TS may be eluted. Delivery system 800 can be deployed by inserting it through ear canal EC and into position in incision IN through tympanic membrane TM with reservoir 810 disposed within tympanic cavity TC. Since, in this embodiment, delivery system 800 is configured similar to the form of a conventional tympanostomy tube, it may be conveniently delivered with the TDS of the '774 patent, optionally including formation and dilation of incision IN in tympanic membrane TM.

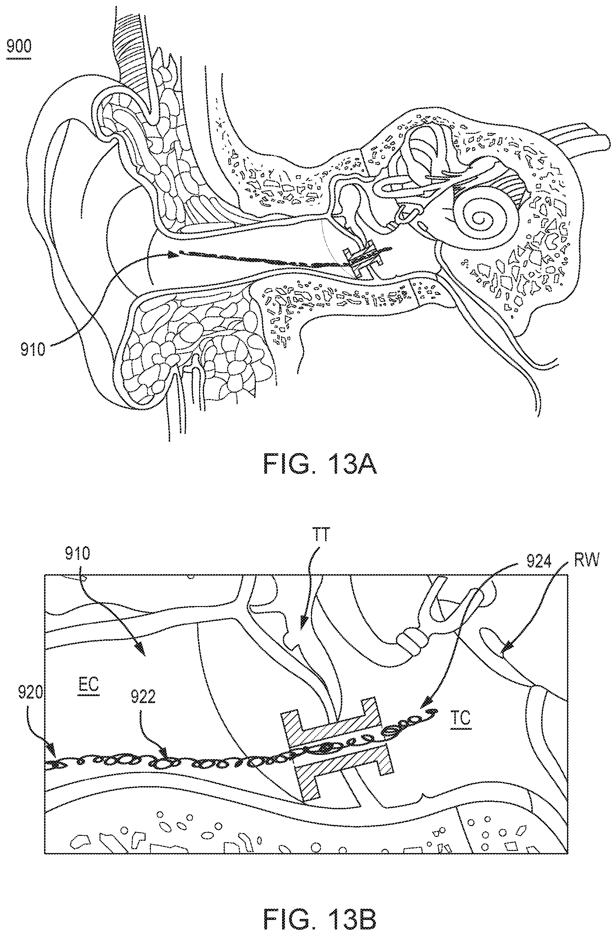

FIGs. 13A and 13B schematically illustrate a delivery system 900 according to another embodiment. In this embodiment, as discussed above, the outer ear OE, and in particular the ear canal EC, functions as the reservoir 910 for the therapeutic substance TS. Outlet 920 is implemented as a wick 922, or set of wicking fibers, that extend from the ear canal, through the central lumen of a tympanostomy tube TT, and into tympanic cavity TC. Alternatively, the wick can extend directly through an incision IS in tympanic membrane TM, rather than through a tympanostomy tube TT. Ear canal EC can be filled, and refilled, with therapeutic substance TS with any suitable device, such as a syringe. Therapeutic substance TS is carried by capillary forces along wick from ear canal EC into tympanic cavity TC. Delivery system 900 can be deployed by inserting it through ear canal EC and into position extending through tympanostomy tube TT or incision IN through tympanic membrane TM with distal end 924 of wick 922 disposed within tympanic cavity TC.

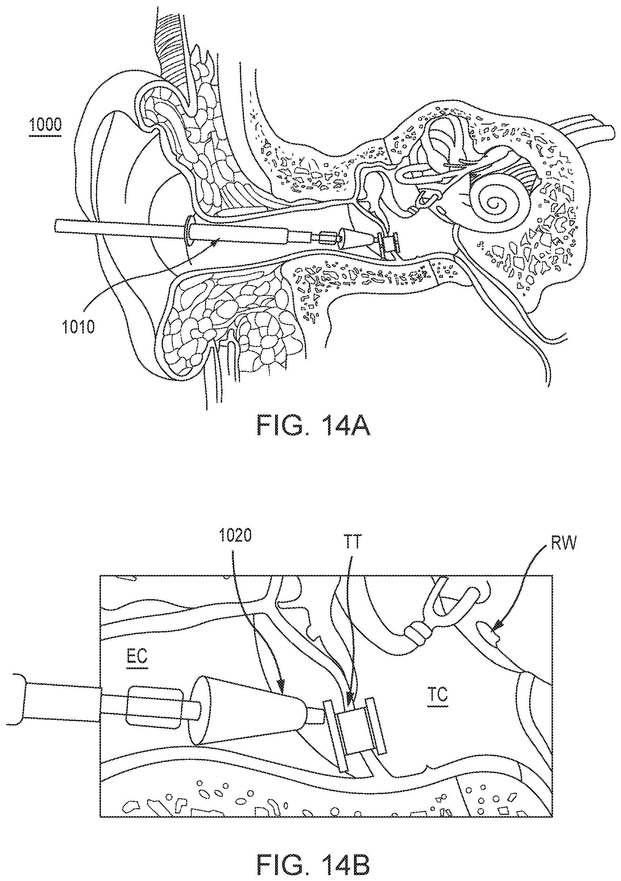

FIGs. 14A and 14B schematically illustrate a delivery system 1000 according to another embodiment. In this embodiment, delivery system 1000 is essentially a syringe, with a variable volume reservoir 1010 within the syringe, and an outlet 1020 at the distal end of the reservoir. Outlet 1020 can be disposed in fluid communication with tympanic cavity TC via the central lumen in tympanostomy tube TT (as shown in FIGs. 14A and 14B), or alternatively directly through incision IN in tympanic membrane TM (not shown). A user can manipulate the syringe to push a piston distally through a barrel forming reservoir 1010 and expel therapeutic substance TS through outlet 1020 and into tympanic cavity TC. Delivery system 1000 can be deployed by inserting it through ear canal EC and into position in engagement with a tympanostomy tube TT that has been delivered, or in engagement with an incision IN that has been formed, in a prior procedure, or as part of the disclosed method.

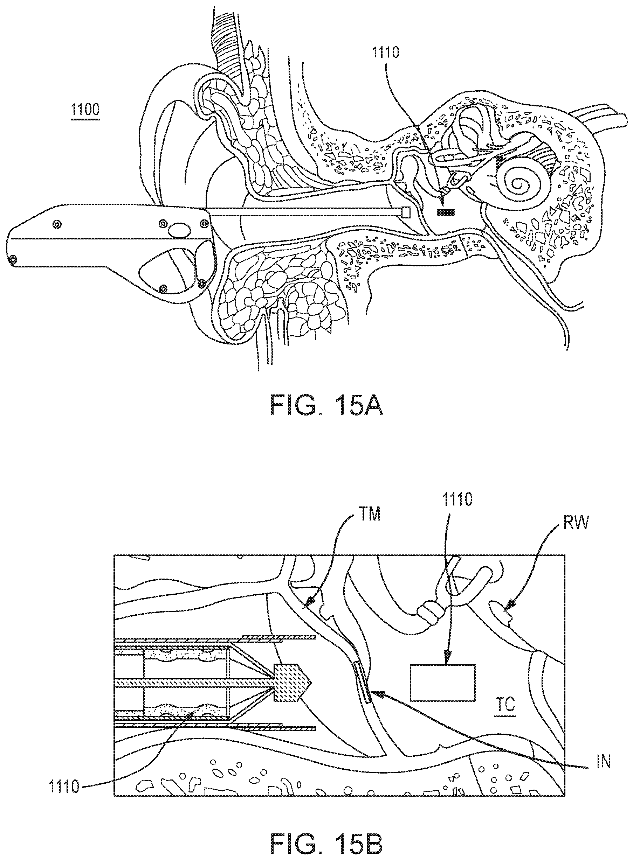

FIGs. 15A and 15B schematically illustrate a delivery system 1100 according to another embodiment. In this embodiment, reservoir 1110 is implemented as a solid, such as a solid dosage form of therapeutic substance TS, a biodegradable material impregnated with therapeutic substance TS, and/or of a material from which therapeutic substance TS may be eluted. Delivery system 1100 can be deployed by depositing reservoir 1110 entirely within tympanic cavity TC through incision IN in tympanic membrane TE. Reservoir 1110 may be conveniently delivered with the TDS of the '774 patent, as shown in FIGs. 15A and 15B, including formation and dilation of incision IN in tympanic membrane TM. Reservoir 1110 may be delivered instead of a tympanostomy tube (as shown in FIG. 15B. with 1110 indicating the reservoir in TDS before deployment, and in tympanic cavity TC after deployment). Reservoir 1110 may also be delivered before or after delivery of a tympanostomy tube.

### Conclusion

While various embodiments have been described and illustrated herein, those of ordinary skill in the art will readily envision a variety of other means and/or structures for perfomling the function and/or obtaining the results and/or one or more of the advantages described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the present disclosure.

The acts performed as part of the method may be ordered in any suitable way. Accordingly, embodiments may be constructed in which acts are performed in an order different than illustrated, which may include performing some acts simultaneously, even though shown as sequential acts in illustrative embodiments.

All definitions, as defined and used herein, should be understood to control over dictionary definitions, definitions in documents referred to herein, and/or ordinary meanings of the defined terms.

The indefinite articles "a" and "an," as used herein in the specification and in the claims, unless clearly indicated to the contrary, should be understood to mean "at least one."

The phrase "and/or." as used herein in the specification and in the claims, should be understood to mean "either or both" of the elements so conjoined. i.e.. elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements
specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of" will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of." "only one of" or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example. "at least one of A and B" (or, equivalently. "at least one of A or B," or, equivalently "at least one of A and/or B") can refer, in one embodiment to at least one, optionally including more than one, A, with no B present (and optionally including elements other than B); in another embodiment, to at least one, optionally including more than one. B, with no A present (and optionally including elements other than A); in yet another embodiment to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," and the like are to be understood to be open-ended. i.e., to mean including but not limited to. Only the transitional phrases "consisting of and "consisting essentially of" shall be closed or semi-closed transitional phrases, respectively.

## Claims

1. A system (400) for delivering a therapeutic substance to at least one of a tympanic cavity or an inner ear in a subject the system comprising:
a reservoir (410) configured to contain the therapeutic substance, the reservoir configured to be disposed within an ear canal of the subject;
a tympanostomy tube configured to be disposed in a tympanic membrane bordering the tympanic cavity
an outlet (420) configured to transport the therapeutic substance from the reservoir to at least one of:
the tympanic cavity; and
a round window to the inner ear bordering the tympanic cavity;
via a lumen of the tympanostomy tube, such that the therapeutic substance is deliverable to at least one of the tympanic cavity and the inner ear.

2. The system of claim 1, wherein the outlet includes at least one of a conduit or a wick.

3. The system of claim 1, wherein the outlet is integral with the tympanostomy tube.

4. The system of any one of claims 1-3, wherein the outlet includes a flow restriction mechanism configured to control a delivery rate of the therapeutic substance into at least one of the lumen of the tympanostomy tube, the tympanic cavity, and the round window.

5. The system of any one of claims 1-4, further comprising an inlet in fluid communication with the reservoir and configured to transport the therapeutic substance to the reservoir.

6. The system of any one of claims 1-5, wherein the reservoir is a bladder having an elastic wall configured to impose a pressure on the therapeutic substance to expel the therapeutic substance from the reservoir through the outlet.

7. The system of any one of claims 1-6, the tympanostomy tube having a proximal end, a distal end, and the lumen extending from the proximal end to the distal end, the tympanostomy tube configured for deployment in the tympanic membrane such that the distal end is positioned in the tympanic cavity, the outlet configured to extend through the lumen of the tympanostomy tube into the tympanic cavity.

8. The system of claim 1, wherein:
the tympanostomy tube has a proximal end and a distal end, the tympanostomy tube configured for deployment in a tympanic membrane bordering the tympanic cavity such that the distal end is positioned in the tympanic cavity;
a solid composite including a polymer and the therapeutic substance, the solid composite disposed on at least the distal end of the tympanostomy tube such that the therapeutic substance can be eluted in the tympanic cavity.

9. The system of claim 8, wherein the solid composite is an adherent layer.

10. The system of claim 8, wherein at least a portion of the tympanostomy tube is formed of the solid composite.

## Patentansprüche

1. Ein System (400) zur Zuführung einer therapeutischen Substanz zu mindestens einem von einer Paukenhöhle oder einem Innenohr bei einem Individuum, wobei das System Folgendes umfasst:
ein Reservoir (410), das dazu ausgelegt ist, die therapeutische Substanz zu enthalten, wobei das Reservoir dazu ausgelegt ist, innerhalb eines Ohrkanals des Individuums angeordnet zu sein;
ein Tympanotomieröhrchen, das dazu ausgelegt ist, in einem Trommelfell, das an die Paukenhöhle angrenzt, angeordnet zu sein;
einen Auslass (420), der dazu ausgelegt ist, die therapeutische Substanz von dem Reservoir zu mindestens einem der Folgenden zu transportieren:
der Paukenhöhle; und
einem runden Fenster zum Innenohr, das an die Paukenhöhle angrenzt;
über ein Lumen des Tympanotomieröhrchens, sodass die therapeutische Substanz zu mindestens einem von der Paukenhöhle und dem Innenohr zugeführt werden kann.

2. System nach Anspruch 1, wobei der Auslass mindestens eines von einer Rohrleitung oder einem Docht umfasst.

3. System nach Anspruch 1, wobei der Auslass mit dem Tympanotomieröhrchen integriert ist.

4. System nach einem der Ansprüche 1-3, wobei der Auslass einen Durchflussbegrenzungsmechanismus einschließt, der dazu ausgelegt ist, eine Zuführungsrate der therapeutischen Substanz in mindestens eines von dem Lumen des Tympanotomieröhrchens, der Paukenhöhle und dem runden Fenster zu steuern.

5. System nach einem der Ansprüche 1-4, das ferner einen Einlass umfasst, der in Fluidverbindung mit dem Reservoir steht und dazu ausgelegt ist, die therapeutische Substanz zu dem Reservoir zu transportieren.

6. System nach einem der Ansprüche 1-5, wobei das Reservoir eine Blase mit einer elastischen Wand ist, die dazu ausgelegt ist, einen Druck auf die therapeutische Substanz auszuüben, um die therapeutische Substanz durch den Auslass aus dem Reservoir auszutreiben.

7. System nach einem der Ansprüche 1-6, wobei das Tympanotomieröhrchen ein proximales Ende, ein distales Ende aufweist und sich das Lumen von dem proximalen Ende zu dem distalen Ende erstreckt, wobei das Tympanotomieröhrchen zum Ablegen in dem Trommelfell ausgelegt ist, sodass das distale Ende in der Paukenhöhle positioniert ist, wobei der Auslass dazu ausgelegt ist, sich durch das Lumen des Tympanotomieröhrchens in die Paukenhöhle zu erstrecken.

8. System nach Anspruch 1, wobei:
das Tympanotomieröhrchen ein proximales Ende und ein distales Ende aufweist, das Tympanotomieröhrchen zum Ablegen in einem Trommelfell ausgelegt ist, das an die Paukenhöhle angrenzt, sodass das distale Ende in der Paukenhöhle positioniert ist;
ein fester Verbundstoff ein Polymer und die therapeutische Substanz einschließt, wobei der feste Verbundstoff auf mindestens dem distalen Ende des Tympanotomieröhrchens angeordnet ist, sodass die therapeutische Substanz in die Paukenhöhle eluiert werden kann.

9. System nach Anspruch 8, wobei der feste Verbundstoff eine haftende Schicht ist.

10. System nach Anspruch 8, wobei mindestens ein Anteil des Tympanotomieröhrchens aus dem festen Verbundstoff gebildet ist.

## Revendications

1. Système (400) destiné à délivrer une substance thérapeutique à au moins l'une d'une cavité tympanique ou d'une oreille interne chez un sujet, le système comprenant :
un réservoir (410) configuré pour contenir la substance thérapeutique, le réservoir configuré pour être disposé au sein d'un conduit auditif du sujet ;
un tube de tympanostomie configuré pour être disposé dans une membrane tympanique bordant la cavité tympanique
une sortie (420) configurée pour transporter la substance thérapeutique du réservoir vers au moins l'un des éléments suivants :
la cavité tympanique ; et
une fenêtre ronde vers l'oreille interne bordant la cavité tympanique ; via une lumière du tube de tympanostomie, de sorte que la substance thérapeutique peut être délivrée à au moins l'une de la cavité tympanique et de l'oreille interne.

2. Système selon la revendication 1, dans lequel la sortie inclut au moins l'un d'un conduit ou d'une mèche.

3. Système selon la revendication 1, dans lequel la sortie est d'un seul tenant avec le tube de tympanostomie.

4. Système selon l'une quelconque des revendications 1 à 3, dans lequel la sortie inclut un mécanisme de restriction d'écoulement configuré pour commander un débit de délivrance de la substance thérapeutique jusque dans au moins l'une de la lumière du tube de tympanostomie, de la cavité tympanique, et de la fenêtre ronde.

5. Système selon l'une quelconque des revendications 1 à 4, comprenant en outre une entrée en communication fluidique avec le réservoir et configurée pour transporter la substance thérapeutique vers le réservoir.

6. Système selon l'une quelconque des revendications 1 à 5, dans lequel le réservoir est une vessie ayant une paroi élastique configurée pour imposer une pression sur la substance thérapeutique pour expulser la substance thérapeutique du réservoir par la sortie.

7. Système selon l'une quelconque des revendications 1 à 6, le tube de tympanostomie ayant une extrémité proximale, une extrémité distale, et la lumière s'étendant de l'extrémité proximale à l'extrémité distale, le tube de tympanostomie configuré pour un déploiement dans la membrane tympanique de sorte que l'extrémité distale est positionnée dans la cavité tympanique, la sortie configurée pour s'étendre à travers la lumière du tube de tympanostomie jusque dans la cavité tympanique.

8. Système selon la revendication 1, dans lequel :
le tube de tympanostomie a une extrémité proximale et une extrémité distale, le tube de tympanostomie configuré pour un déploiement dans une membrane tympanique bordant la cavité tympanique de sorte que l'extrémité distale est positionnée dans la cavité tympanique ;
un composite solide incluant un polymère et la substance thérapeutique, le composite solide disposé sur au moins l'extrémité distale du tube de tympanostomie de sorte que la substance thérapeutique peut être éluée dans la cavité tympanique.

9. Système selon la revendication 8, dans lequel le composite solide est une couche adhérente.

10. Système selon la revendication 8, dans lequel au moins une partie du tube de tympanostomie est formée du composite solide.
